# EUROPEAN PATENT APPLICATION

(11) **EP 4 571 290 A1**
(43) Date of publication of application: **18.06.2025**
(21) Application number: 23877198.4
(22) Date of filing: 03.10.2023
(51) Int. Cl.: G01N 17/00

(54) **METHOD FOR EVALUATING DELAYED FRACTURE PROPERTIES OF METAL MATERIAL, METHOD FOR SELECTING METAL MATERIAL, AND METHOD FOR MANUFACTURING MEMBER**

(30) Priority: 13.10.2022 JP 2022164400
(71) Applicant: JFE Steel Corporation, Tokyo 100-0011 (JP)
(72) Inventor: NOZAKI, Ayaka, Tokyo 100-0011 (JP); OTSUKA, Shinji, Tokyo 100-0011 (JP); MORIMOTO, Minako, Tokyo 100-0011 (JP); HATA, Kentaro, Tokyo 100-0011 (JP)
(74) Representative: Grünecker Patent- und Rechtsanwälte PartG mbB
(86) International application number: PCT/JP2023/036125
(87) International publication number: WO 2024/080193

(57) **Abstract**

A method for evaluating delayed fracture characteristics of a metal material is provided that is capable of accurately evaluating characteristics regarding delayed fracture induced by hydrogen having penetrated into the metal material as a result of atmospheric corrosion and that can simulate delayed fracture characteristics in an actual use environment.

The method for evaluating delayed fracture characteristics of a metal material includes performing at least one or more times steps including the following step (A) and step (B). Step (A): a step including a chloride attaching step (a1) of attaching a chloride to the metal material in an atmosphere having a relative humidity Hal of 80% or more and a temperature Tal of 60°C or below. Step (B): a step of performing one or more times a cycle including a predetermined drying step (b1), a predetermined wetting step (b2), a predetermined transition step (b3), and a predetermined transition step (b4).

## Description

### Technical Field

The present invention relates to a method for evaluating delayed fracture characteristics of a metal material. In particular, the present invention pertains to a method for evaluating delayed fracture characteristics (such as the occurrence or absence of delayed fracture, and the degree of delayed fracture) of a metal material for use in an atmospheric corrosive environment in which delayed fracture is induced by hydrogen that penetrates into the metal material as a result of corrosion.

### Background Art

In recent years, lightweight automobile structural members have been explored through efforts to reduce the thickness of steel sheets that are used, by increasing their strength. This strengthening of steel sheets has given rise to a new concern about delayed fracture that has not been encountered in conventional automobile parts.

Delayed fracture is a phenomenon in which a high-strength steel part is suddenly fractured in a brittle way with almost no apparent plastic deformation after being stressed by a static load for a certain time. In a broad sense, this fracture includes, for example, liquid metal contact cracking and stress corrosion cracking (Non Patent Literature 1). A problem that can occur in automobile parts is delayed fracture by hydrogen embrittlement that is induced by hydrogen having penetrated into the steel as a result of corrosion. It is known that delayed fracture is caused by three factors: material (strength), working (strain and stress), and hydrogen. Here, the hydrogen that penetrates into metal materials can come from solutions and solvents that are brought into contact with the metal materials, or can be generated as a result of the metal materials being corroded in use environments.

In the past, delayed fracture has been extensively studied in the field of plates, such as line pipes, that absorb a large amount of hydrogen from solutions and solvents, and in the field of high-strength steel bolts that achieve a tensile strength of 1200 MPa or more (Non Patent Literature 2). Methods for evaluating delayed fracture characteristics in these fields have been standardized.

On the other hand, automobile parts are used in atmospheric environments. Thus, proper evaluation of delayed fracture characteristics of automobile parts critically depends on the establishment of a method for evaluating characteristics regarding delayed fracture by hydrogen embrittlement that is induced by hydrogen having penetrated into automobile parts as a result of atmospheric corrosion. To address this, methods for evaluating delayed fracture characteristics by simulating an atmospheric corrosive environment have been proposed.

For example, Patent Literature 1 discloses a method for evaluating delayed fracture characteristics of a metal material by performing one or more times steps consisting of a step of attaching a chloride-based component to the metal material, and a step of performing at least one time a cycle in which the surface of the metal material is dried and wetted by changing the relative humidity around the metal material.

Patent Literature 2 discloses a method for evaluating hydrogen embrittlement characteristics that includes a salt attaching step of attaching a metal salt including a chloride to the surface of a metal material; and a basic step including performing one or more times a basic cycle including one wetting step in which the metal material is exposed to an atmosphere having a relative humidity Hh and one drying step in which the metal material is exposed to an atmosphere having a relative humidity Hlo (with the proviso that Hlo < Hh), wherein the salt attaching step, the wetting step, and the drying step are performed in an atmosphere of 40°C or below, and at least one drying step in the basic step is performed in an atmosphere having a temperature of 30°C or below, at a relative humidity of 0% to 60%, for a period of 1 minute or more and 6 hours or less.

### Citation List

### Patent Literature

PTL 1: Japanese Unexamined Patent Application Publication No. 2016-180658
PTL 2: International Publication No. 2019/186940

### Non Patent Literature

NPL 1: Okurehakai (Delayed Fracture), Shinsaku MATSUYAMA, NIKKAN KOGYO SHIMBUN, LTD., 1989
NPL 2: Omura et al., JSCE symposium handout, vol. 170, pp. 47-54, 2010
NPL 3: Corrosiveness of various atmospheric test sites as measured by specimens of steel and zinc, in Metal Corrosion in the Atmosphere, STP 435, pp. 360-391, American Society for Testing and Materials, Philadelphia, 1968

### Summary of Invention

### Technical Problem

One of the parameters in the atmospheric corrosive environment that significantly affect delayed fracture characteristics of metal materials is temperature.

In the evaluation method described in Patent Literature 1, the temperature in the test steps is constant and the evaluation method can evaluate the range of temperatures that affect delayed fracture characteristics. The present inventors have evaluated delayed fracture characteristics of high-strength steel sheets by the evaluation method described in Patent Literature 1. The results have shown that the method sometimes fails to reproduce delayed fracture characteristics in an actual use environment (in a real environment).

The evaluation method described in Patent Literature 2 involves varied temperatures in the test steps and is therefore incapable of accurately evaluating the influence of temperature on delayed fracture characteristics of metal materials. Furthermore, the evaluation method does not anticipate evaluating delayed fracture characteristics in a high-temperature environment above 40°C.

The present invention has been made in view of the circumstances discussed above. It is therefore an object of the present invention to provide a method for evaluating delayed fracture characteristics of a metal material that is capable of accurately evaluating characteristics regarding delayed fracture induced by hydrogen having penetrated into the metal material as a result of atmospheric corrosion during use of the metal material in an atmospheric corrosive environment and that can simulate delayed fracture characteristics in an actual use environment.

### Solution to Problem

The present inventors carried out extensive studies and have found that the above object can be achieved by adopting the following configurations, thus completing the present invention.

[1] A method for evaluating a delayed fracture characteristic of a metal material, including performing at least one or more times steps including a step (A) and a step (B) described below:
   step (A): a step including a chloride attaching step (a1) of attaching a chloride to the metal material in an atmosphere having a relative humidity Ha1 of 80% or more and a temperature Ta1 of 60°C or below;
   step (B): a step of performing at least one or more times a cycle including a drying step (b1), a wetting step (b2), a transition step (b3), and a transition step (b4) described below, the cycle being performed in an atmosphere having a temperature Tb1 that is 60°C or below and falls in a certain range,
   drying step (b1): a step of drying the metal material by holding the metal material in an atmosphere having a relative humidity Hb1 of 45% or less for 1.0 hour or more and 5.0 hours or less;
   wetting step (b2): a step of wetting the metal material by holding the metal material in an atmosphere having a relative humidity Hb2 of 80% or more for 1.0 hour or more and 5.0 hours or less;
   transition step (b3): a step of changing the atmosphere having the relative humidity Hb1 to the atmosphere having the relative humidity Hb2 in 1.0 hour or more and 5.0 hours or less;
   transition step (b4): a step of changing the atmosphere having the relative humidity Hb2 to the atmosphere having the relative humidity Hb1 in 1.0 hour or more and 5.0 hours or less.
[2] The method for evaluating a delayed fracture characteristic of a metal material according to [1], wherein the step (A) further includes, after the chloride attaching step (a1), a holding step (a2) of holding the metal material in the atmosphere having the relative humidity Ha1 and the temperature Ta1.
[3] The method for evaluating a delayed fracture characteristic of a metal material according to [2], wherein a holding time in the holding step (a2) is set so that a process time of the step (A) is equal to a process time of the wetting step (b2).
[4] The method for evaluating a delayed fracture characteristic of a metal material according to any one of [1] to [3], wherein the metal material has a coating layer on a surface.
[5] A method for selecting a metal material, including:
   an evaluation step of evaluating a delayed fracture characteristic of a metal material using the method for evaluating a delayed fracture characteristic of a metal material described in any one of [1] to [4]; and
   a selection step of selecting a metal material based on an evaluation result obtained in the evaluation step.
[6] A method for manufacturing a member, including working a metal material selected by the method for selecting a metal material described in [5] to produce a member.

### Advantageous Effects of Invention

The method for evaluating delayed fracture characteristics of a metal material according to the present invention is capable of accurately evaluating characteristics regarding delayed fracture induced by hydrogen having penetrated into the metal material as a result of atmospheric corrosion during use of the metal material in an atmospheric corrosive environment and can simulate delayed fracture characteristics in an actual use environment.

Furthermore, the method for evaluating delayed fracture characteristics of a metal material according to the present invention can provide information necessary to determine whether a delayed fracture will occur in a metal material in an actual use environment, for example, the fracture limit stress at which delayed fracture will occur depending on an actual use environment.

### Brief Description of Drawings

[Fig. 1] Fig. 1 is a diagram illustrating an embodiment of corrosion test cycles according to a method for evaluating delayed fracture characteristics of a metal material of the present invention.
[Fig. 2] Fig. 2 is a view schematically illustrating a test specimen for the evaluation of delayed fracture characteristics used in Examples.

### Description of Embodiments

First, the findings by the present inventors will be described. Patent Literature 1 describes a method for evaluating characteristics regarding delayed fracture induced by hydrogen having penetrated into a metal material as a result of atmospheric corrosion during use of the metal material in an atmospheric corrosive environment. The present inventors evaluated delayed fracture characteristics of commercial 1470 MPa grade cold rolled steel sheets (thickness: 1.4 mm) under the corrosion test cycle conditions described below conforming to the method of Patent Literature 1 while changing the stress that was applied among YS (yield stress) equivalent, 1000 MPa, 800 MPa, 600 MPa, and 400 MPa.

Specifically, a commercial 1470 MPa grade cold rolled steel sheet (thickness: 1.4 mm) was sheared to 35 mm in width × 100 mm in length, and was ground to a width of 30 mm to remove the residual stress from shearing. Rectangular test specimens were thus prepared. The rectangular test specimens obtained were immersed in toluene and ultrasonically cleaned for 5 minutes, and were then bent 180° with a radius of curvature of 4 mm R. The test specimens were restrained in this state with bolts and nuts to fix the shape of the test specimens. In order to change the stress that was applied, the inner spacings of the rectangular test specimens after the bending were 8, 10, 12, 14, and 17 mm. The test was performed for 63 days at maximum. The test specimens were observed every day to check for the presence or absence of cracking. Furthermore, the same test specimens were evaluated for delayed fracture characteristics in a real environment (Okinawa) (an exposure test (1 year)). The results are described in Table 1.

### (Corrosion test cycle conditions)

### [Step of attaching a chloride to the metal materials]

·Air atmosphere
·Chloride: NaCl
·Amount attached: 10 g/m²
·Method of attaching: spraying

### [Step of performing one or more times a cycle including a drying step and a wetting step]

·Drying step conditions: temperature 30°C, humidity 30% RH, holding time 2 hours
·Transition time from the drying step to the wetting step: 2 hours (excluding the holding time in the humidity keeping step described below), humidity change rate: 30% RH/hr
·Wetting step conditions: temperature 30°C, humidity 90% RH, holding time 2 hours
·Transition time from the wetting step to the drying step: 2 hours (excluding the holding time in the humidity keeping step described below), humidity change rate: 30% RH/hr
·Humidity keeping step conditions: 55% RH, holding time 2 hours
·Cycle order: Drying step → Humidity keeping step → Wetting step → Humidity keeping step → Drying step

**[Table 1]**

| Testing methods | Applied stress/MPa*¹ | | | | |
|---|---|---|---|---|---|
| | YS equivalent | 1000 | 800 | 600 | 400 |
| Method described in Patent Literature 1 | 5/5 | 4/5 | 0/5 | 1/5 | 0/5 |
| Exposure test in actual environment (Okinawa) | 5/5 | 4/5 | 0/5 | 0/5 | 0/5 |

| | | | | | |
|---|---|---|---|---|---|
| *1 The denominator is the number of test specimens tested, and the numerator is the number of test specimens cracked. | | | | | |

From Table 1, the method described in Patent Literature 1 had variability in the evaluation results of delayed fracture characteristics, and the occurrence and the absence of cracking varied depending on the test specimens when the load stress to the test specimen was low (when the stress applied was 600 MPa). In other words, it can be said that the method described in Patent Literature 1 sometimes fails to reproduce delayed fracture characteristics in a real environment. The present inventors conducted a detailed investigation into the cause of this failure. As a result, the present inventors have found that in the method described in Patent Literature 1, cracks occur sometimes in the metal material in the step of attaching a chloride-based component to the metal material (the chloride attaching step). While the mechanism by which cracks occur in the metal material in the chloride attaching step is not clearly understood, the speculation of the present inventors is as follows.

In an atmospheric corrosive environment (a real environment), a chloride becomes attached to a metal material as a result of attachment and accumulation of snow-melting salts and airborne salt. The chloride absorbs moisture to form a water film on the metal material, and consequently the metal material is corroded (atmospheric corrosion). As the corrosion progresses, hydrogen generated by the corrosion penetrates into the metal material, giving rise to cracks. Unfortunately, it is difficult to reproduce the manner of chloride attachment in a real environment by experiments. In experiments, the amount of chloride attached to a metal material is approximated to that in a real environment by, for example, atomizing or spraying a solution (salt water) having a controlled chloride concentration to the surface of the metal material or by immersing the metal material into such a salt water. In this manner, the salt water is applied so that the desired amount of chloride will be attached. The salt water is generally applied in an air atmosphere.

When the salt water is applied as described above, the relative humidity at least in the vicinity of the surface of the metal material changes from the relative humidity of the air atmosphere to nearly 100% relative humidity. When a chloride has been attached to the surface of the metal material, the chloride absorbs moisture and forms a water film in which the chloride is dissolved. Furthermore, the application of the salt water forms a thick salt water film. After the application of the salt water has been completed, the relative humidity near the surface of the metal material changes to the relative humidity of the air atmosphere, and the water film begins to dry. The water film decreases its thickness and the chloride is concentrated in the water film. When the relative humidity near the surface of the metal material falls below the chloride deliquescence relative humidity, the chloride is precipitated and the water film almost disappears.

At a level near the chloride deliquescence relative humidity, the water film contains an increased amount of chloride ions and metal ions dissolved by corrosion. The pH of the water film decreases as the water film changes its thickness along with the change in relative humidity from wet to dry, and atomic hydrogen forms on the surface of the metal material and more hydrogen penetrates into the metal material. Thus, the amount of hydrogen inside the metal material is increased by repeating of the chloride attaching step in an air atmosphere. As a result, the amount of hydrogen that penetrates into the metal material exceeds the fracture limit amount of hydrogen even under conditions where cracks do not occur in a real environment. The present inventors speculate that cracks in the metal material stemmed from the reasons above. Thus, the present inventors believe that improving the accuracy of the evaluation of delayed fracture characteristics critically depends on reducing the amount of hydrogen that penetrates into the metal material in the chloride attaching step, and have carried out various studies, thereby completing the present invention. The present invention has been made based on the above findings.

A method for evaluating delayed fracture characteristics of a metal material according to the present invention (hereinafter, also written simply as the evaluation method of the present invention) includes performing at least one or more times steps including a step (A) and a step (B).

The evaluation method of the present invention preferably involves applying a stress to the metal material. For example, the stress may be applied to the metal material by working the metal material (working method). Examples of the working methods include bending, bulging, stretching, and twisting. Alternatively, for example, the metal material may be restrained in a stressed shape using a bolt or the like, or residual stress that remains after working may be used. In the evaluation method of the present invention, steps including a step (A) and a step (B) may be performed at least one or more times (one time, or two or more times) on a metal material stressed in the manner described above. In the evaluation method of the present invention, the condition of the metal material is checked after the steps including the step (A) and the step (B) are performed at least one or more times, and delayed fracture characteristics of the metal material can be evaluated based on the condition of the metal material that has been checked. For example, the condition may be checked by observing, for example, visually observing for the presence or absence of cracking in the metal material, the degree of cracking, and the like.

The evaluation method of the present invention will be described below with reference to an exemplary embodiment. However, the present invention is not limited to the exemplary embodiment below.

### (Metal materials)

First, a metal material that is subjected to the method for evaluating delayed fracture characteristics of a metal material of the present embodiment (hereinafter, also written simply as the evaluation method of the present embodiment) will be described. For example, the metal material may be a steel material, but is not limited thereto and may be such a metal material as Ti or Al. The metal material may have a coating layer on a surface. Examples of the coating layers include organic layers, inorganic layers, and mixture layers of an organic substance and an inorganic substance. The coating layer may be a single layer or a multilayer. For example, the metal material having a coating layer on a surface may be a metal material in which the surface of the metal material is chemical conversion treated, for example, zinc phosphate treated, and a film layer is further formed on the chemical conversion layer by electrodeposition coating or the like.

The form of the metal material is not particularly limited and may be, for example, sheet, bar, or tube. However, the evaluation method of the present embodiment is suited for the evaluation of a sheet material or a tube material having a relatively small thickness, and it is therefore preferable that the evaluation workpiece be a sheet material or a tube material.

In the evaluation method of the present embodiment, stress is applied to the metal material. For example, the stress may be applied to the metal material in the manner described hereinabove.

In the present embodiment, the metal material under stress is subjected to a step (A) and a step (B) in order to evaluate delayed fracture characteristics of the metal material. After each of these steps is performed one or more times, delayed fracture characteristics are evaluated by checking for the presence or absence of cracking in the metal material, and the degree of cracking.

The steps will be individually described below.

### (Step (A))

The step (A) is a step including a chloride attaching step (a1) of attaching a chloride to the metal material in an atmosphere having a relative humidity Ha1 of 80% or more and a temperature Ta1 of 60°C or below.

### [Chloride attaching step (a1)]

In the chloride attaching step (a1), a chloride is attached to the metal material so as to obtain a desired amount of the chloride attached. The chloride preferably includes one, or two or more selected from sodium salt (NaCl), potassium salt (KCl), calcium salt (CaCl₂), and magnesium salt (MgCl₂) that exist in the atmospheric environment in which general metal materials are used. The chloride that is attached to the metal material in the chloride attaching step (a1) may be a chloride-based component that includes a chloride and a component other than chlorides. Here, the chloride-based component is a component in which a chloride represents more than 50 mass% of the whole component in terms of solid content. Examples of the components other than chlorides include, but are not limited to, sulfides and nitric acid compounds. In consideration of an actual atmospheric corrosive environment, it is preferable to attach a NaCl-based component (a component in which NaCl represents more than 50 mass% of the whole component) to the metal material.

When simulating delayed fracture characteristics in a region where a snow-melting agent is frequently spread in winter, it is preferable that the component that is attached to the metal material have a composition similar to that of the snow-melting agent spread in that region. Examples of the components having a composition similar to that of snow-melting agents include CaCl₂-based components (components in which CaCl₂ represents more than 50 mass% of the whole component), MgCl₂-based components (components in which MgCl₂ represents more than 50 mass% of the whole component), and NaCl-based components (components in which NaCl represents more than 50 mass% of the whole component).

Furthermore, a component containing a combination of metal salts may be attached to the metal material. Examples of the components containing a combination of metal salts include SAE (Society of Automotive Engineers, Inc.) J2334 (0.5 mass% NaCl-0.1 mass% CaCl₂-0.075 mass% NaHCO₃), and artificial seawater (containing 2.5 mass% NaCl-0.5 mass% MgCl₂-0.12 mass% CaCl₂-0.07 mass% KCl (for example, an aqueous solution of Aquamarine (registered trademark) manufactured by YASHIMA PURE CHEMICALS CO., LTD.)).

The amount in which the chloride is attached to the metal material (the amount of the solid chloride excluding solvent, such as water) is preferably 1 to 100000 mg/m². This amount attached corresponds to the expected amount of chloride attached in an actual atmospheric corrosive environment. In a corrosive environment in which the amount attached is less than 1 mg/m², corrosion scarcely proceeds. Thus, the metal material can be evaluated for delayed fracture characteristics but the evaluation takes time due to the low corrosion rate. Furthermore, the amount of hydrogen generated by the corrosion is so small that delayed fracture is unlikely to occur, and in many cases the evaluation of delayed fracture characteristics itself is not necessary. Thus, the amount attached is preferably 1 mg/m² or more. When, on the other hand, the amount attached is more than 100000 mg/m², there is a high risk that an actual corrosive environment cannot be simulated. Thus, the amount attached is preferably 100000 mg/m² or less. From the points of view of simulating the form of corrosion in an actual atmospheric corrosive environment and of accelerating the corrosion, the amount attached is more preferably 100 to 30000 mg/m².

The method of attaching the chloride to the metal material (the chloride attaching method) is not particularly limited. A chloride attaching method of usual choice is to attach a solution containing a chloride as solute to the metal material. Specific examples include a method in which the metal material is immersed into a solution containing a chloride-based component (usually an aqueous solution, such as salt water, hereinafter also written as salt water), and a method in which salt water is atomized or sprayed to the metal material.

The chloride concentration in the salt water is not particularly limited. When, however, the chloride is attached to the metal material using salt water having a chloride concentration of less than 0.1 mass%, it takes time to obtain a preferred amount of chloride attached or it is difficult to obtain a preferred amount of chloride attached. Thus, the chloride concentration in the salt water is preferably 0.1 mass% or more, and more preferably 1 mass% or more. When, on the other hand, the chloride is attached (the salt water is applied) to the metal material by, for example, atomizing or spraying salt water with a chloride concentration of 20 mass% or more in the salt water, the chloride is easily precipitated in the salt water atomizing or spraying device and, for example, will clog the device, making it difficult to apply salt water with a stable concentration. In addition, it becomes difficult to uniformly attach the chloride to the metal material. As a result, the amount of chloride attached to the metal material varies from place to place. Corrosion occurs locally in areas where the amount of chloride attached is large, and a larger amount of hydrogen penetrates. Thus, delayed fracture characteristics vary within the evaluation region of the metal material, and the accuracy of the evaluation of delayed fracture characteristics is lowered. In particular, this tendency is noticeable when the amount of chloride attached is large. Thus, the chloride concentration in the salt water is preferably 20 mass% or less, and more preferably 15 mass% or less.

The time for which the salt water is applied to the metal material to attach the chloride, namely, the process time of the chloride attaching step (a1) is not particularly limited. The process time of the chloride attaching step (a1) is preferably 0.2 hours or more. The process time of the chloride attaching step (a1) is preferably 1.0 hour or less.

When the process time of the chloride attaching step (a1) is less than 0.2 hours, the amount of chloride attached may have nonuniform distribution over the surface of the metal material, and delayed fracture characteristics may vary within the evaluation region of the surface of the metal material. Thus, the process time of the chloride attaching step (a1) is preferably 0.2 hours or more. The process time of the chloride attaching step (a1) is more preferably 0.3 hours or more. When, on the other hand, the process time of the chloride attaching step (a1) is more than 1.0 hour, the corrosion behavior may differ from that in an actual environment. Specifically, a phenomenon may occur in which corrosion products formed on the surface of the metal material are washed away together with the salt water being supplied to the surface of the metal material. When, for example, the metal material is a Zn-coated steel sheet, the corrosion products effectively block the penetration of hydrogen into the base steel sheet. Thus, washing away of the corrosion products together with the salt water eliminates the effects of the corrosion products in an actual environment and also invites a risk that corrosion due to the salt water will proceed. As a result, the correlation with corrosion in an actual environment is lowered and the results of delayed fracture characteristics may not agree with results in an actual environment. Thus, the duration of the chloride attaching step (a1) is preferably 1.0 hour or less, and more preferably 0.7 hours or less.

The amount of chloride attached may be calculated by multiplying the difference in mass of the test specimen (the metal material) before and after the attachment of chloride in the chloride attaching step (a1) by the chloride concentration in the salt water, and dividing the product by the area of the test specimen. The amount of chloride attached may be controlled by, for example, changing the chloride concentration in the salt water or changing the time for which the salt water is applied (the process time of the chloride attaching step (a1)) so as to change the amount of the salt water applied and attached to the metal material.

When the salt water is applied to the surface of the metal material by atomization, the inclination angle of the surface of the metal material may be, for example, 0° with respect to the horizontal plane. Because in this case a water film of the salt water accumulates on the surface of the metal material, the surface may be inclined with respect to the horizontal plane. The inclination angle of the surface of the metal material may be similarly controlled also in steps other than the chloride attaching step (a1). That is, the inclination angle of the surface of the metal material may be set to 0° with respect to the horizontal plane or may be inclined also in steps other than the chloride attaching step (a1). By inclining the inclination angle of the metal material with respect to the horizontal plane also in steps other than the chloride attaching step (a1), the chloride is washed away and new chloride is easily attached in the chloride attaching step (a1).

### <Relative humidity Ha1 in the chloride attaching step (a1): 80% or more>

In the chloride attaching step (a1), the chloride is attached to the metal material in an atmosphere having a relative humidity Ha1 of 80% or more. When the chloride is attached to the metal material by atomization or spraying in the chloride attaching step (a1), the relative humidity of the environment near the surface of the metal material rises to nearly 100%. The same applies also when the metal material is immersed in the salt water. When, for example, the chloride is attached to the metal material in an air atmosphere, the relative humidity in the environment after the completion of attaching the chloride falls below 80% and the chloride is concentrated in the water film on the surface of the metal material. Consequently, an increased amount of hydrogen penetrates into the metal material in such a chloride attaching step. In the evaluation method of the present invention, the chloride is attached to the metal material under conditions where the relative humidity Ha1 in the chloride attaching step (a1) is 80% or more. By virtue of this configuration, the relative humidity of the environment after the chloride has been attached does not become less than 80%. The relative humidity is away from the vicinity of the chloride deliquescence relative humidity, and the chloride is not concentrated in the water film. Thus, the amount of hydrogen that penetrates into the metal material in the chloride attaching step (a1) can be reduced. The upper limit of the relative humidity Ha1 in the chloride attaching step (a1) is not particularly limited. For example, the relative humidity Ha1 may be less than 98%, may be 95% or less, or may be 90% or less.

### <Temperature Ta1 in the chloride attaching step (a1): 60°C or below>

In the chloride attaching step (a1), the chloride is attached to the metal material in an atmosphere having a temperature Ta1 of 60°C or below. When the temperature Ta1 in the chloride attaching step (a1) is above 60°C, the evaluation takes place in an environment not close to a corrosive environment in which the metal material is actually used, and further the corrosion mechanism will alter. Thus, the temperature Ta1 in the chloride attaching step (a1) is limited to 60°C or below, and is preferably 50°C or below. On the other hand, the lower limit of the temperature Ta1 in the chloride attaching step (a1) is not particularly limited. When the temperature Ta1 in the chloride attaching step (a1) is below 5°C, it may be difficult to control the relative humidity in a corrosion test tank (a constant temperature and humidity tank) used in the corrosion test. Furthermore, the corrosion of the metal material is significantly retarded and the evaluation time is extended. Thus, the temperature Ta1 in the chloride attaching step (a1) is preferably 5°C or above, and more preferably 10°C or above.

### [Holding step (a2)]

The step (A) preferably further includes a holding step (a2) where the metal material to which the chloride has been attached in the chloride attaching step (a1) is held in the atmosphere having the relative humidity Ha1 and the temperature Ta1. As a result of the metal material being held at the relative humidity Ha1 after the chloride attaching step (a1), the thickness of the water film formed by the application of salt water is allowed to change to the thickness of a water film that will be formed by the moisture absorption of the chloride at the relative humidity Ha1. As a result, it is expected that the water film that is formed has a uniform chloride concentration over the surface of the metal material, and the unevenness in the amount of chloride attached to the surface of the metal material is effectively eliminated. Thus, it is preferable that the chloride attaching step (a1) be followed by the holding step (a2) of holding the metal material in the atmosphere having the relative humidity Ha1 and the temperature Ta1. The duration of the holding step (a2), that is, the holding time at the relative humidity Ha1 is preferably 0.5 hours or more. The upper limit of the holding time is not particularly limited. The holding time in the holding step (a2) is preferably set so that the process time of the step (A) (here, the total duration of the chloride attaching step (a1) and the holding step (a2)) is equal to the process time of a wetting step (b2) in the step (B) described later. By thus setting the process time of the step (A), the step (A) can replace a wetting step (b2) in a cycle of the step (B) as will be described later, and regular corrosion test cycles can be constructed easily. Furthermore, by setting the relative humidity Ha1 to the same relative humidity as the relative humidity Hb2 in the wetting step (b2) described later, the amount of hydrogen that is caused to penetrate into the metal material by the chloride attaching step (a1) can be further reduced. Thus, it is preferable that the relative humidity Ha1 be set to the same relative humidity as the relative humidity Hb2 in the wetting step (b2) described later.

The amount of hydrogen that penetrates into a metal material as a result of corrosion of the metal material in an atmospheric corrosive environment varies greatly depending on the temperature of the environment (the atmosphere). Thus, delayed fracture characteristics too are strongly affected by the temperature of the environment. In order to appropriately evaluate delayed fracture characteristics of the metal material taking into consideration the role of a metal material member (a member made of the metal material) and the environment in which the metal material member is used, it is therefore preferable that the temperature of the atmosphere be kept constant in the step (A) (here, the chloride attaching step (a1), or the chloride attaching step (a1) and the holding step (a2)) and in the step (B) described later. A change in atmosphere temperature can occur during the chloride attaching step (a1) probably due to the influence of water that is applied, and difficulties are sometimes encountered in strictly controlling the temperature of the atmosphere. However, the influence on delayed fracture characteristics can be significantly reduced when the variation of the temperature Ta1 of the atmosphere in the step (A) can be controlled to within ±10°C relative to the temperature Tb1 of the atmosphere in the step (B). Thus, it is preferable that the variation of the temperature Ta1 of the atmosphere in the step (A) be controlled to within ±10°C relative to the temperature Tb1 of the atmosphere in the step (B).

Atmospheric corrosive environments are characterized in that they alternate between wet (wet condition) and dry (dry condition). Simulating such environmental changes is important in approximating the form of corrosion found in real environments. In the case of, for example, steel materials, it is known that corrosion products that occur on the steel materials change depending on whether the condition is wet or dry, and that hydrogen is generated in the process where wetness dries or dryness is wetted. For this reason, the conditions in a cycle involving a change in relative humidity (step (B)) are also important in evaluating delayed fracture characteristics.

### (Step (B))

The step (B) is a step of performing at least one or more times (one time, or two or more times) a cycle including a drying step (b1), a wetting step (b2), a transition step (b3), and a transition step (b4) described below. The cycle is performed in an atmosphere having a temperature Tb1 that is 60°C or below and falls in a certain range.

### <Temperature Tb1 of the step (B): 60°C or below and within a certain range>

The step (B) is performed in an atmosphere having a temperature Tb1 that is 60°C or below and falls in a certain range. When the temperature Tb1 in the step (B) is above 60°C, the evaluation takes place in an environment not close to a corrosive environment in which the metal material is actually used, and further the corrosion mechanism will alter. Thus, the temperature Tb1 in the step (B) is limited to 60°C or below, and is preferably 50°C or below. On the other hand, the lower limit of the temperature Tb1 in the step (B) is not particularly limited. When the temperature Tb1 in the step (B) is below 5°C, it may be difficult to control the relative humidity in a corrosion test tank (a constant temperature and humidity tank) used in the corrosion test. Furthermore, the corrosion of the metal material is significantly retarded and the evaluation time is extended. Thus, the temperature Tb1 in the step (B) is preferably 5°C or above, and more preferably 10°C or above.

As already mentioned, delayed fracture characteristics are strongly affected by the temperature of the environment (the atmosphere). In order to appropriately evaluate delayed fracture characteristics of the metal material taking into consideration the role of a metal material member and the environment in which the metal material member is used, it is therefore necessary that the temperature Tb1 in the step (B) be controlled to a certain range. When the variation in the temperature Tb1 in the step (B) is within ±5°C, the amount of hydrogen that penetrates from the environment into the metal material (the amount of hydrogen penetration) can be evaluated with a variation of within 30% relative to the amount of hydrogen penetration at the target environmental temperature, and delayed fracture characteristics can be accurately evaluated. When the variation in the temperature Tb1 in the step (B) is within ±2°C, the variation of the amount of hydrogen penetration is within 15%. Thus, the variation in the temperature Tb1 in the step (B) is preferably within ±5°C, and more preferably within ±2°C.

### [Drying step (b1)]

The drying step (b1) is a step of drying the metal material by holding the metal material in an atmosphere having a relative humidity Hb1 of 45% or less for 1.0 hour or more and 5.0 hours or less. The relative humidity Hb1 in the drying step (b1) is limited to 45% or less in order to simulate a dry condition that is one of the characteristics of atmospheric corrosive environments. When the relative humidity Hb1 in the drying step (b1) is above 45%, a long holding time is necessary to sufficiently dry the surface of the metal material and the evaluation time is extended. The relative humidity Hb1 in the drying step (b1) is preferably 40% or less. On the other hand, the lower limit of the relative humidity Hb1 in the drying step (b1) is not particularly limited. From the point of view of the controllability of the relative humidity, the relative humidity Hb1 in the drying step (b1) is preferably 20% or more. When the component that is attached to the surface of the metal material includes a substance that deliquesces at a lower relative humidity, such as magnesium chloride or calcium chloride, it is preferable that the relative humidity Hb1 in the drying step (b1) be set low.

The process time of the drying step (b1) (the time for which the metal material is held in an atmosphere having the relative humidity Hb1) is limited to 1.0 hour or more and 5.0 hours or less. When the process time of the drying step (b1) is less than 1.0 hour, an actual corrosive environment cannot be simulated. When, on the other hand, the process time of the drying step (b1) is more than 5.0 hours, an actual corrosive environment can be simulated but the evaluation of delayed fracture characteristics takes a long time.

### [Wetting step (b2)]

The wetting step (b2) is a step of wetting the metal material by holding the metal material in an atmosphere having a relative humidity Hb2 of 80% or more for 1.0 hour or more and 5.0 hours or less. The relative humidity Hb2 in the wetting step (b2) is limited to 80% or more in order to simulate a wet condition that is one of the characteristics of atmospheric corrosive environments. When the relative humidity Hb2 in the wetting step (b2) is less than 80%, the effect of wetting is insufficient and, as a result, an actual corrosive environment cannot be simulated. Among the chlorides, sodium chloride has the highest critical saturated vapor pressure. The critical saturated vapor pressure of sodium chloride is about 75 to 78% in terms of relative humidity. Thus, any chloride absorbs moisture at a relative humidity of 80% or more to form a water film that keeps wet the surface of the metal material. Thus, the relative humidity Hb2 in the wetting step (b2) is limited to 80% or more. On the other hand, the upper limit of the relative humidity Hb2 in the wetting step (b2) is not particularly limited. It is, however, preferable that the relative humidity Hb2 in the wetting step (b2) be less than 98%. When the relative humidity Hb2 is 98% or more, the thickness of the water film is excessively increased by condensation, and the chloride that has been attached tends to be washed away. This phenomenon is likely to occur particularly when the test specimen that is evaluated has been worked. Thus, the relative humidity Hb2 in the wetting step (b2) is preferably controlled to less than 98% when evaluating a worked test specimen.

The process time of the wetting step (b2) (the time for which the metal material is held in an atmosphere having a relative humidity Hb2 of 80% or more) is limited to 1.0 hour or more and 5.0 hours or less. When the process time of the wetting step (b2) is less than 1.0 hour, an actual corrosive environment cannot be simulated. When, on the other hand, the process time of the wetting step (b2) is more than 5.0 hours, an actual corrosive environment can be simulated but the evaluation of delayed fracture characteristics takes a long time.

### [Transition step (b3) and transition step (b4)]

The transition step (b3) is a step of changing the atmosphere having the relative humidity Hb1 to the atmosphere having the relative humidity Hb2. The transition step (b4) is a step of changing the atmosphere having the relative humidity Hb2 to the atmosphere having the relative humidity Hb1. It is known that the amount of hydrogen that penetrates into a metal material, in particular a steel material, increases at a timing when the relative humidity changes. That is, a large amount of hydrogen penetrates into the steel material in the transition step (b3) and the transition step (b4). As an example, the transition step (b4) will be discussed in which the atmosphere in the wetting step (b2) having the relative humidity Hb2 transitions to the atmosphere in the drying step (b1) having the relative humidity Hb1. In the wetting step (b2), the chloride deliquesces to form a water film with a water film thickness in accordance with the relative humidity in the environment, and corrosion of the metal material proceeds. In the drying step (b1), on the other hand, the chloride in most cases does not deliquesce. The chloride that does not deliquesce is precipitated and the water film almost disappears, leaving almost no progress in corrosion. In the presence of such a change in water film thickness, chloride ions and metal ions dissolved by corrosion are concentrated in the water film when the relative humidity is around the chloride deliquescence relative humidity, and hydrolysis reaction occurs in the chloride-concentrated water film. As a result, the pH of the water film decreases, and atomic hydrogens form on the surface of the metal material. Thus, an increased amount of hydrogen penetrates into the metal material. In the transition step (b3), which is the reverse of the transition step (b4), a chloride-concentrated water film is formed in the vicinity of the chloride deliquescence relative humidity, but the amount of metal ions dissolved by corrosion is relatively small compared with that in the transition step (b4). Thus, the amount of hydrogen penetration in the transition step (b3) is smaller than that in the transition step (b4). Here, in order to ensure a sufficient reaction time for the hydrolysis and to ensure the amount of hydrogen penetration, the process time of the transition step (b3) and that of the transition step (b4) need to be each 1.0 hour or more, and are preferably each 1.5 hours or more. On the other hand, the process time of the transition step (b3) and that of the transition step (b4) are each limited to 5.0 hours or less. When these process times are longer than 5.0 hours, the chloride concentrating rate slows down and the progress of the hydrolysis reaction becomes slow. Such an evaluation method takes a long time to evaluate delayed fracture characteristics and is not appropriate.

When the chloride includes NaCl, the amount of hydrogen that penetrates into the metal material is largest in a region where the relative humidity is near the NaCl deliquescence humidity, specifically, 55% or more and 75% or less. In this region, however, the metal material does not substantially undergo corrosion. Thus, either or both of the transition step (b3) and the transition step (b4) may include a holding step in which the metal material is held in a region where the relative humidity is 55% or more and 75% or less for a predetermined time (a holding step at a relative humidity of 55% or more and 75% or less). By providing the holding step, the amount of hydrogen that penetrates into the metal material can be increased. The process time of the holding step is preferably 0.5 hours or more. The process time of the transition step (b3) and that of the transition step (b4) include the process time of the holding step at a relative humidity of 55% or more and 75% or less.

The method for evaluating delayed fracture characteristics of a metal material according to the present invention aims to simulate a change in relative humidity between day and night in a real environment. When the process time of the step (B) (the duration of one cycle) simulating a change in relative humidity between day and night in a real environment is longer than 24 hours, the corrosion is slower than in a real environment and the evaluation of delayed fracture characteristics takes a long time. That is, it is preferable to set the process time of the step (B) to 24 hours or less. In order to expedite the evaluation, it is more preferable to set the process time of the step (B) to 12 hours or less. On the other hand, shortening the process time of the step (B) increases the sharpness of the change in relative humidity and may lower the correlation with corrosion in a real environment, and the results may not agree with delayed fracture characteristics in a real environment. Thus, the process time of the step (B) is preferably set to 5 hours or more.

In the evaluation method of the present invention, the step (A) and the step (B) are each performed at least one time. For the convenience for simulating the form of corrosion in an actual environment and setting the corrosion test cycles, the step (A) is preferably incorporated into a cycle of the step (B) in place of the wetting step (b2) as will be described later. The step (A) may be introduced for every random number of cycles of the step (B) or for every predetermined number of cycles of the step (B). The upper limit of the number of times the steps including the step (A) and the step (B) are performed is not particularly limited. For example, the steps including the step (A) and the step (B) may be performed until cracking occurs in the metal material. Alternatively, the number of test days may be determined beforehand and the steps may be performed a number of times corresponding to the number of test days. The number of times the steps are performed may be determined appropriately taking into consideration factors, such as, for example, the simulation of the form of corrosion in an actual environment. As an example, the steps may be performed 1500 times or less. The number of times the steps are performed may be determined in accordance with factors, such as the quality and the type of the metal material to be evaluated. For example, the upper limit of the number of times may be set to 1500 times when delayed fracture characteristics are evaluated with respect to a metal material having a coating layer on the surface, and the upper limit of the number of times may be set to 420 times when evaluating delayed fracture characteristics of a metal material having no coating layer on the surface.

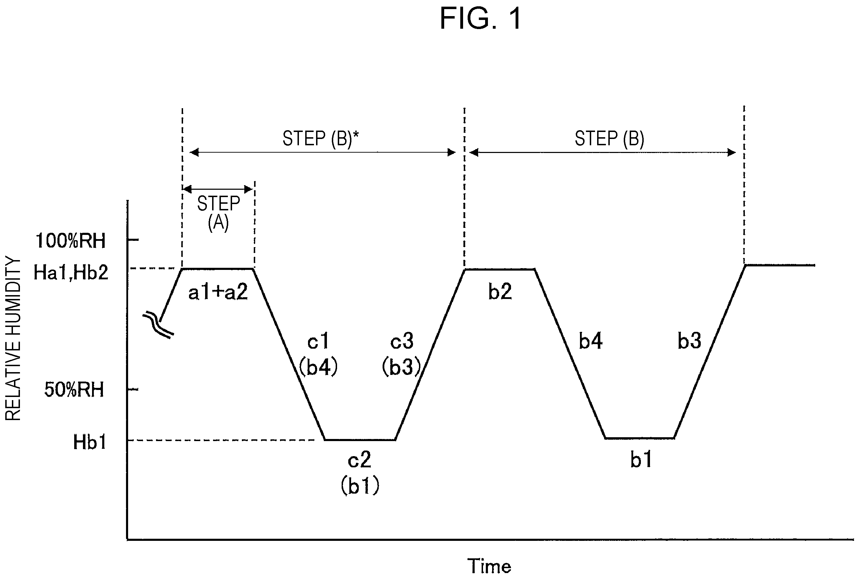

Next, the steps including the step (A) and the step (B) will be described. Fig. 1 is a diagram illustrating an embodiment of corrosion test cycles according to the evaluation method of the present invention. The corrosion test cycle illustrated in Fig. 1 represents an example of a corrosion test cycle in which the step (A) and the step (B) are each performed one time. In this example, the step (A) includes a chloride attaching step (a1) and a holding step (a2). The step (B) includes a cycle composed of a wetting step (b2), a transition step (b4), a drying step (b1), and a transition step (b3).

The corrosion test cycle illustrated in Fig. 1 includes a step (a step (B)*) of performing a cycle in which the step (A) is followed by a step of changing the atmosphere of the step (A) to an atmosphere having a relative humidity Hb1 (a transition step (c1)), a step of holding the metal material in the atmosphere having a relative humidity Hb1 (a holding step (c2)), and a step of changing the atmosphere having a relative humidity Hb1 to an atmosphere having a relative humidity Hb2 (a transition step (c3)).

In the present embodiment, the step (B)* is set as a cycle corresponding to the cycle of the step (B) except that the step (A) is introduced in place of the wetting step (b2). That is, the process time of the step (A) in the step (B)* (the total process time of the chloride attaching step (a1) and the holding step (a2)) is set equal to the process time of the wetting step (b2) in the step (B). Furthermore, the transition step (c1), the holding step (c2), and the transition step (c3) in the step (B)* are set to the same conditions as the transition step (b4), the drying step (b1), and the transition step (b3) in the step (B), respectively. As a result, the corrosion test cycle illustrated in Fig. 1 is set so that the process time of the step (B)* and the process time of the step (B) are equal to each other. By introducing the step (A) into the cycle of the step (B) in place of the wetting step (b2) as described above, regular corrosion test cycles are constructed easily. Furthermore, such corrosion test cycles can simulate the penetration of hydrogen stemming from actual atmospheric corrosion while simulating an actual corrosive environment (an atmospheric corrosive environment) more faithfully.

In the evaluation method of the present embodiment, the corrosion test cycle may be set so that the steps including the step (B)* and the step (B) are performed at least one or more times (one time, or two or more times). The step (B)* may be introduced for every predetermined number of cycles of the step (B) or for every random number of cycles of the step (B). In the evaluation method of the present embodiment, the corrosion test cycle may be set so that the steps consisting of the step (B)* and the step (B) are performed at least one or more times.

In the corrosion test cycle illustrated in Fig. 1, the transition step (c1), the holding step (c2), and the transition step (c3) in the step (B)* are set to the same conditions as the transition step (b4), the drying step (b1), and the transition step (b3) in the step (B), respectively. However, the configuration is not limited thereto. For example, the transition step (c1) may have different conditions, such as process time, from the transition step (b4) in the step (B). For example, the process time of the transition step (c1) (the transition time) may be less than 1.0 hour. Similarly, the transition step (c3) may have different conditions, such as process time, from the transition step (b3) in the step (B). For example, the process time of the transition step (c3) may be less than 1.0 hour.

In the corrosion test cycle illustrated in Fig. 1, the step (A) includes the chloride attaching step (a1) and the holding step (a2). However, the configuration is not limited thereto. The step (A) may be free of the holding step (a2).

The corrosion test cycle illustrated in Fig. 1 includes the step in which the step (A) is followed by the transition step (c1), the holding step (c2), and the transition step (c3) (the step (B)*). However, the configuration is not limited thereto. The corrosion test cycle may be such that the step (A) is immediately followed by the step (B) without the transition step (c1), the holding step (c2), and the transition step (c3). The cycle of the step (B) may start from the wetting step (b2) or from other step (such as the transition step (b4)).

In the evaluation method of the present invention, the steps including the step (A) and the step (B) described above are performed at least one or more times, the metal material is checked for condition (such as the presence or absence of cracking in the metal material, and the degree of cracking), and delayed fracture characteristics of the metal material are evaluated based on the condition of the metal material that has been checked.

A method for selecting a metal material according to the present invention includes an evaluation step of evaluating delayed fracture characteristics of a metal material using the method for evaluating delayed fracture characteristics of a metal material described hereinabove, and a selection step of selecting a metal material based on an evaluation result obtained in the evaluation step. Performing the evaluation step on a stressed metal material gives a relationship between the corrosion test conditions (such as temperature, relative humidity, and the amount of chloride attached) and delayed fracture characteristics (such as the occurrence or absence of delayed fracture and the degree of delayed fracture (for example, the test time to the occurrence of cracking)). Furthermore, metal materials under different levels of stress may be tested under the same corrosion test condition or conditions to obtain a relationship, and the metal materials can be classified based on the relationship. An appropriate metal material, for example, a metal material to be shipped, can be selected from the classified metal materials depending on the environment in which the metal material will be used, and the level of stress that will be applied to members.

A method for manufacturing a member according to the present invention includes a step of working a metal material selected by the method for selecting a metal material described hereinabove to produce a member. The working is not particularly limited and may be any metal working, such as forming. The member is preferably an automobile member.

### EXAMPLES

The present invention will be described below by presenting Examples. However, the present invention is not limited to the following Examples.

### (Metal materials)

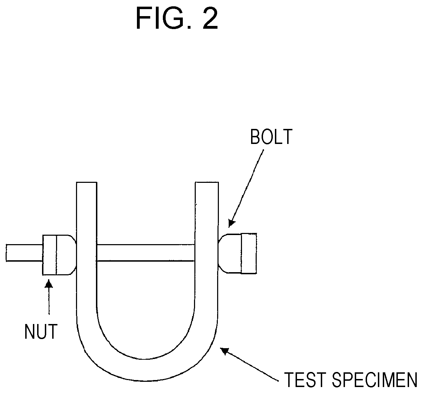

Commercial 1.4 mm thick 1470 MPa grade cold rolled steel sheets and the same cold rolled steel sheets having a coating layer on the surface were provided. These metal materials were evaluated for delayed fracture characteristics according to Inventive Examples and Comparative Examples. The surface-coated cold rolled steel sheets were produced as follows. First, the surface of the cold rolled steel sheet was degreased with alkali and was then subjected to a zinc phosphate conversion treatment to form a chemical conversion layer (coating weight: 2 g/m²). Electrodeposition coating was applied to the chemical conversion layer to form a film layer (film thickness: 15 µm). PB-SX35 manufactured by Nihon Parkerizing Co., Ltd. was used as the chemical conversion agent in the zinc phosphate conversion treatment. GT-100 manufactured by Kansai Paint Co., Ltd. was used as the electrodeposition paint in the electrodeposition coating. The cold rolled steel sheets and the surface-coated cold rolled steel sheets as the workpieces were each sheared to 35 mm in width × 100 mm in length and ground to a width of 30 mm to remove the residual stress from shearing. Rectangular test specimens (steel sheets) were thus produced. Among the rectangular test specimens obtained, those having no coating layer on the surface were immersed in toluene and were ultrasonically cleaned for 5 minutes. This cleaning was not performed for those having a coating layer on the surface. The test specimens were bent 180° and were restrained in the bent state with a bolt and a nut to fix the shape of the test specimen. Test specimens for the evaluation of delayed fracture characteristics illustrated in Fig. 2 were thus obtained. The test specimens for the evaluation of delayed fracture characteristics (hereinafter simply written as the "test specimens" for descriptive convenience) included (i) those obtained by bending the rectangular test specimen (steel sheet) 180° with a radius of curvature of 4 mm R so that the inner spacing of the bent rectangular test specimen was 8 mm, and (ii) those obtained by bending the rectangular test specimen (steel sheet) 180° with a radius of curvature of 5 mm R so that the inner spacing of the bent rectangular test specimen was 10 mm.

### (Corrosion test cycles)

The test specimens described above were subjected to a corrosion test cycle (a corrosion test) consisting of the following step (B)* and step (B). Details of the conditions of the corrosion test cycle are described in Table 2-1 and Table 2-2. The step (B) is a step in which wetting step (b2) → transition step (b4) → drying step (b1) → transition step (b3) are performed in this order, and the completion of the above four steps counts as one cycle. The step (B)* differs from the step (B) in that the wetting step (b2) is replaced by the step (A). Specifically, the step (B)* is a step in which step (A) [chloride attaching step (a1) → holding step (a2)] → transition step (b4) → drying step (b1) → transition step (b3) are performed in this order. The evaluation of delayed fracture characteristics was made according to the following corrosion test cycles (I) to (IV). The symbol **"-"** in Table 2-1 and Table 2-2 means that the step was not performed.
Cycles (I): Cycles in which step (B)* → step (B) are repeated.
Cycles (II): Cycles in which step (B)* → step (B) → step (B) → step (B) are repeated.
Cycles (III): Cycles in which cycle (I) → cycle (II) are repeated, specifically, cycles in which step (B)* → step (B) → step (B)* → step (B) → step (B) → step (B) are repeated. Cycles (IV): Cycles in which step (B)* alone is repeated.

### (1) Evaluation of delayed fracture characteristics

Delayed fracture characteristics were evaluated based on the number of days until cracking occurred in the test specimens. Specifically, during the corrosion test, the 180° bent portions of the test specimens were visually observed once a day for the presence or absence of cracking, and the number of days until cracking occurred (the days to cracking) was examined for a maximum of 63 days. Here, cracking was determined to have occurred when a new crack not seen in the surface of the worked portion before the corrosion test grew to 1 mm or more. The corrosion test for evaluating delayed fracture characteristics was performed on three specimens for each Example (Inventive Example or Comparative Example), and the number of days until cracking occurred in two or more specimens was taken as the days to cracking.

The evaluation results of the delayed fracture characteristics are described in Table 3. The results described in Table 3 also include cases that ended with only one specimen cracked.

The same test specimens (test specimens for delayed fracture evaluation) as those used in the corrosion test of this Example were subjected to an exposure test in Okinawa, Japan, conducted by the present inventors. As a result, those test specimens produced from the cold rolled steel sheets were cracked on day 24 when the radius of curvature was 4 mm R, and survived one year of exposure without cracking when the radius of curvature was 5 mm R. Furthermore, those test specimens produced from the surface-coated cold rolled steel sheets were cracked on day 210 when the radius of curvature was 4 mm R, and survived one year of exposure without cracking when the radius of curvature was 5 mm R. The average temperature in the period to the occurrence of cracking in the test specimens with a radius of curvature of 4 mm R was 26°C, and the average temperature in one year of exposure experienced by the test specimens with a radius of curvature of 5 mm R was 20°C. Based on these results, the results of the evaluation of delayed fracture characteristics in the above-described corrosion test were rated as "∘" (excellent accuracy in evaluating delayed fracture characteristics) when cracking had occurred in two or more test specimens with a radius of curvature of 4 mm R and had not occurred at all in the test specimens with a radius of curvature of 5 mm R. Other cases were rated as ''×'' (poor accuracy in evaluating delayed fracture characteristics).

### (2) Evaluation of the quality of the corrosive environment

In the present invention, the ability to simulate an actual use environment (corrosive environment) is important in order to evaluate delayed fracture characteristics under an actual use environment (actual corrosive environment). Whether the corrosion test had well simulated an actual corrosive environment (the quality of the corrosive environment) was examined by evaluating the ratio of the amount of corrosion of a cold rolled steel sheet to that of a zinc block based on the criteria described later. The cold rolled steel sheet and the zinc block used in this evaluation test were a commercial cold rolled steel sheet with low impurity contents (SPCE according to the JIS standard) and a zinc sheet with 99% purity, respectively. The quantitative corrosion ratios that were used as standards were based on the results of the steel to zinc quantitative corrosion ratios obtained by testing, such as an exposure test in an actual environment described in Non Patent Literature 3. A failure of the ratio of the amount of corrosion of the cold rolled steel sheet to that of the zinc block in the corrosion test to fall within the standard quantitative corrosion ratio means that the corrosion test fails to simulate an actual corrosive environment (the environment reproduced is not an actual corrosive environment). In other words, a failure of the ratio of the amount of corrosion of the cold rolled steel sheet to that of the zinc block in the corrosion test to be within the standard quantitative corrosion ratio means that the cold rolled steel sheet (the metal material) is evaluated in a corrosive situation different from an actual corrosive environment, and it is probable that the occurrence of hydrogen that causes delayed fracture will be significantly different from that from corrosion in an actual corrosive environment. It is therefore necessary that the corrosion test simulate an actual corrosive environment.

The amount of corrosion of the cold rolled steel sheet was calculated by dividing the difference in mass before and after the corrosion test (before and after the corrosion) by the corroded area. The mass of the cold rolled steel sheet after the corrosion test was measured after the corrosion products were removed by immersing the cold rolled steel sheet from the corrosion test in 5% HCl solution. Similarly, the amount of corrosion of the zinc block was calculated by dividing the difference in mass before and after the corrosion test by the corroded area, and the mass of the zinc block after the corrosion test was measured after the corrosion products were removed by immersing the zinc block from the corrosion test in ammonium dichromate for 15 minutes. [Cold rolled steel sheet to zinc block quantitative corrosion ratio] in the corrosion test was calculated from the following formula, and the quality of the corrosive environment was evaluated according to the following evaluation criteria. [Cold rolled steel sheet to zinc block quantitative corrosion ratio] = [Amount of corrosion of cold rolled steel sheet]/[Amount of corrosion of zinc block]

### <Evaluation criteria>

o (Suitable corrosive environment): 10 ≤ [Cold rolled steel sheet to zinc block quantitative corrosion ratio] ≤ 100
Δ (Appropriate corrosive environment): 3 ≤ [Cold rolled steel sheet to zinc block quantitative corrosion ratio] < 10, or 100 < [Cold rolled steel sheet to zinc block quantitative corrosion ratio] ≤ 500
× (Inappropriate corrosive environment): [Cold rolled steel sheet to zinc block quantitative corrosion ratio] < 3, or 500 < [Cold rolled steel sheet to zinc block quantitative corrosion ratio]
The corrosion test was determined to have simulated an actual use environment (corrosive environment) when the ratio based on the above evaluation criteria was rated as "∘" or "Δ", and was determined to have failed to simulate an actual use environment when the rating was "x".

The overall rating was "o" (the corrosion test was capable of accurately evaluating characteristics regarding delayed fracture induced by hydrogen having penetrated into the metal material as a result of atmospheric corrosion and was capable of simulating delayed fracture characteristics in an actual use environment) when the evaluation of delayed fracture characteristics (1) was rated as "o" and the quality of the corrosive environment (2) was rated as "∘" or "Δ". Other cases were rated as ''×'' (the corrosion test was poor in the accuracy in evaluating delayed fracture characteristics and/or was incapable of simulating delayed fracture characteristics in an actual use environment).

As described in Table 2-1, Table 2-2, and Table 3, the evaluation method of the present invention can accurately evaluate characteristics regarding delayed fracture induced by hydrogen having penetrated into the metal material as a result of atmospheric corrosion and can simulate delayed fracture characteristics in an actual use environment.

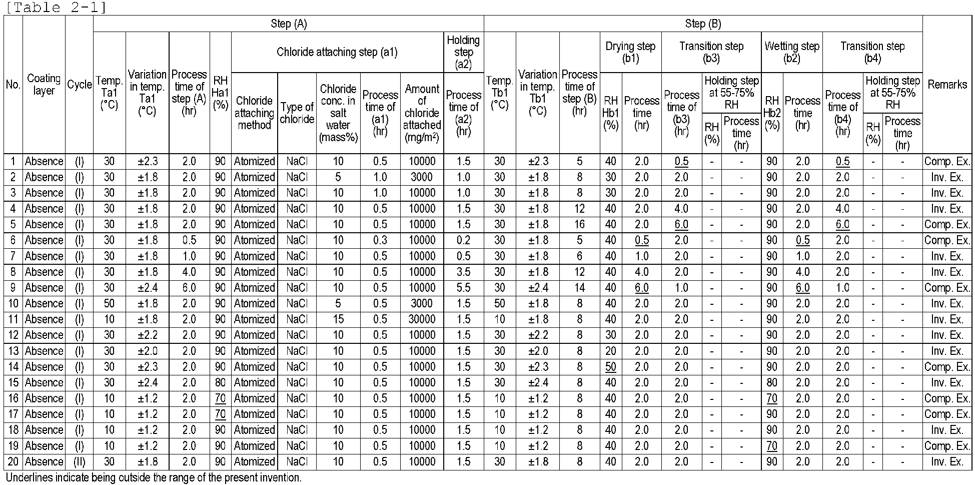

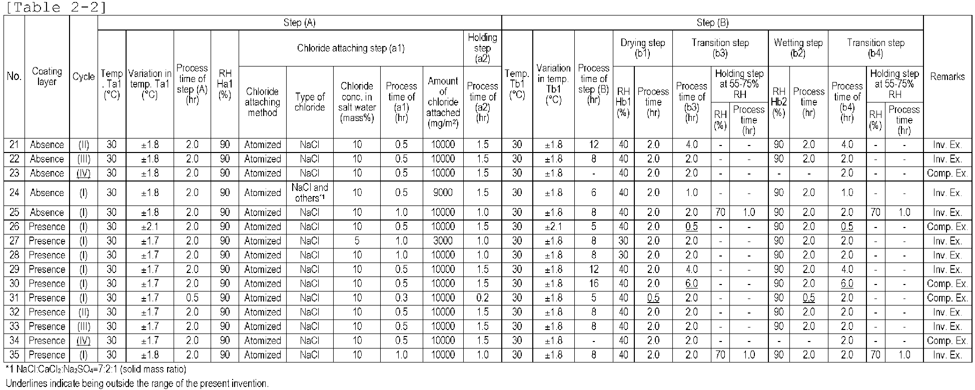

**[Table 3]**

| No. | Delayed fracture characteristics | | | Evaluation of quality of corrosive environment | Overall rating | Remarks |
|---|---|---|---|---|---|---|
| | Days to cracking (days)*¹ | | Rating | | | |
| | Radius of curvature 4mmR | Radius of curvature 5mmR | | | | |
| 1 | No cracking | No cracking | × | × | × | Comp. Ex. |
| 2 | 25 | No cracking | ○ | ○ | ○ | Inv. Ex. |
| 3 | 10 | No cracking | ○ | ○ | ○ | Inv. Ex. |
| 4 | 33 | No cracking | ○ | ○ | ○ | Inv. Ex. |
| 5 | No cracking | No cracking | × | ○ | × | Comp. Ex. |
| 6 | 4 | No cracking | ○ | × | × | Comp. Ex. |
| 7 | 7 | No cracking | ○ | ○ | ○ | Inv. Ex. |
| 8 | 18 | No cracking | ○ | ○ | ○ | Inv. Ex. |
| 9 | No cracking | No cracking | × | ○ | × | Comp. Ex. |
| 10 | 9 | No cracking | ○ | ○ | ○ | Inv. Ex. |
| 11 | 8 | No cracking | ○ | ○ | ○ | Inv. Ex. |
| 12 | 21 | No cracking | ○ | ○ | ○ | Inv. Ex. |
| 13 | 36 | No cracking | ○ | ○ | ○ | Inv. Ex. |
| 14 | 4 | Only one specimen cracked (24) | × | × | × | Comp. Ex. |
| 15 | 13 | No cracking | ○ | ○ | ○ | Inv. Ex. |
| 16 | 5 | Only one specimen cracked (16) | × | × | × | Comp. Ex. |
| 17 | 3 | 20 | × | ○ | × | Comp. Ex. |
| 18 | 20 | No cracking | ○ | ○ | ○ | Inv. Ex. |
| 19 | 10 | Only one specimen cracked (10) | × | × | × | Comp. Ex. |
| 20 | 10 | No cracking | ○ | ○ | ○ | Inv. Ex. |
| 21 | 17 | No cracking | ○ | ○ | ○ | Inv. Ex. |
| 22 | 11 | No cracking | ○ | ○ | ○ | Inv. Ex. |
| 23 | 4 | 22 | × | × | × | Comp. Ex. |
| 24 | 25 | No cracking | ○ | ○ | ○ | Inv. Ex. |
| 25 | 4 | No cracking | ○ | ○ | ○ | Inv. Ex. |
| 26 | No cracking | No cracking | × | × | × | Comp. Ex. |
| 27 | 49 | No cracking | ○ | ○ | ○ | Inv. Ex. |
| 28 | 45 | No cracking | ○ | ○ | ○ | Inv. Ex. |
| 29 | 52 | No cracking | ○ | ○ | ○ | Inv. Ex. |
| 30 | No cracking | No cracking | × | ○ | × | Comp. Ex. |
| 31 | 30 | No cracking | ○ | × | × | Comp. Ex. |
| 32 | 45 | No cracking | ○ | ○ | ○ | Inv. Ex. |
| 33 | 46 | No cracking | ○ | ○ | ○ | Inv. Ex. |
| 34 | 31 | 55 | × | × | × | Comp. Ex. |
| 35 | 28 | No cracking | ○ | ○ | ○ | Inv. Ex. |

| | | | | | | |
|---|---|---|---|---|---|---|
| *1 The number in parentheses is the number of days to cracking in the "one specimen". | | | | | | |

## Claims

1. A method for evaluating a delayed fracture characteristic of a metal material, comprising performing at least one or more times steps comprising a step (A) and a step (B) described below:
step (A): a step comprising a chloride attaching step (a1) of attaching a chloride to the metal material in an atmosphere having a relative humidity Hal of 80% or more and a temperature Tal of 60°C or below;
step (B): a step of performing at least one or more times a cycle comprising a drying step (b1), a wetting step (b2), a transition step (b3), and a transition step (b4) described below, the cycle being performed in an atmosphere having a temperature Tb1 that is 60°C or below and falls in a certain range,
drying step (b1): a step of drying the metal material by holding the metal material in an atmosphere having a relative humidity Hb1 of 45% or less for 1.0 hour or more and 5.0 hours or less;
wetting step (b2): a step of wetting the metal material by holding the metal material in an atmosphere having a relative humidity Hb2 of 80% or more for 1.0 hour or more and 5.0 hours or less;
transition step (b3): a step of changing the atmosphere having the relative humidity Hb1 to the atmosphere having the relative humidity Hb2 in 1.0 hour or more and 5.0 hours or less;
transition step (b4): a step of changing the atmosphere having the relative humidity Hb2 to the atmosphere having the relative humidity Hb1 in 1.0 hour or more and 5.0 hours or less.

2. The method for evaluating a delayed fracture characteristic of a metal material according to claim 1, wherein the step (A) further comprises, after the chloride attaching step (a1), a holding step (a2) of holding the metal material in the atmosphere having the relative humidity Hal and the temperature Tal.

3. The method for evaluating a delayed fracture characteristic of a metal material according to claim 2, wherein a holding time in the holding step (a2) is set so that a process time of the step (A) is equal to a process time of the wetting step (b2).

4. The method for evaluating a delayed fracture characteristic of a metal material according to any one of claims 1 to 3, wherein the metal material has a coating layer on a surface.

5. A method for selecting a metal material, comprising:
an evaluation step of evaluating a delayed fracture characteristic of a metal material using the method for evaluating a delayed fracture characteristic of a metal material described in any one of claims 1 to 4; and
a selection step of selecting a metal material based on an evaluation result obtained in the evaluation step.

6. A method for manufacturing a member, comprising working a metal material selected by the method for selecting a metal material described in claim 5 to produce a member.
